# EUROPÄISCHE PATENTANMELDUNG

(11) **EP 1 574 492 A2**
(43) Veröffentlichungstag der Anmeldung: **14.09.2005**
(21) Anmeldenummer: 04025627.3
(22) Anmeldetag: 28.10.2004
(51) Int. Cl.: C07C 2/36, C07C 11/107

(54) **Verfahren zur Darstellung von 1-Hexen und Katalysatorvorstufe**

(30) Priorität: 12.03.2004 DE 102004012107
(71) Anmelder: Forschungszentrum Karlsruhe GmbH, 76133 Karlsruhe (DE)
(72) Erfinder: Döring, Manfred, Prof. Dr., 76744 Wörth-Büchelberg (DE); Bluhm, Martin, Dr., 68723 Oftersheim (DE)

(57) **Zusammenfassung**

Der Erfindung liegt die Aufgabe zugrunde, ein Verfahren zur Darstellung von 1-Hexen und eine dabei einsetzbare Katalysatorvorstufe vorzuschlagen. Es wird ein Verfahren beschrieben, bei dem Ethen in Gegenwart einer Katalysatorvorstufe, bei der Chrom mit einem Liganden, der Phosphor und Stickstoff enthält, komplexiert ist, und in Gegenwart von Methylaluminiumoxan als Co-Katalysator zu 1-Hexen umgesetzt wird, das dadurch gekennzeichnet ist, dass die Katalysatorvorstufe durch die folgende chemische Formel beschrieben wird, 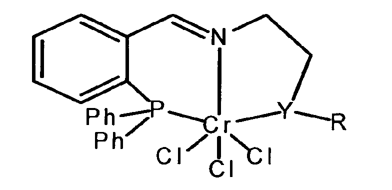 wobei Y entweder Phosphor oder Schwefel darstellt und R entweder Aryl- oder Alkylreste sind.

## Beschreibung

Die Erfindung betrifft ein Verfahren zur Darstellung von 1-Hexen gemäß Anspruch 1 und eine Katalysatorvorstufe gemäß Anspruch 2.

In der Veröffentlichung "*First Cr(III)-SNS Complexes and Their Use as Highly Efficient Catalysts for the Trimerisation of Ethylene to 1-Hexene"* von David S. MCGuinness et al. in J. AM. CHEM. SOC: **2003,** 125, 5272-5273 wird ein Katalysatorsystem für die Trimerisierung von Ethen zu 1-Hexen beschrieben, das aus einem Chrom(III)-Komplex mit dreizähnigen PNP-Liganden und einem Co-Katalysator besteht. Der Co-Katalysator ist Methylaluminoxan (MAO).

Die Herstellung dieses Co-Katalysators und seine Verwendung sind auch in "Novel Cr-PNP complexes as catalysts for the trimerisation of ethylene" von David S. McGuinness et al. in CHEM. COMMUN., 2003, 334-335 veröffentlicht.

Obwohl diese Katalysatorvorstufe zusammen mit Methylaluminoxan die Reaktion von Ethen zu 1-Hexan wirkungsvoll und selektiv katalysiert, besteht doch das Problem, dass die Darstellung der Katalysatorvorstufe verhältnismäßig aufwendig ist.

Der Erfindung liegt die Aufgabe zugrunde, eine weitere Katalysatorvorstufe und ein Herstellungsverfahren für 1-Hexen vorzuschlagen, wobei die Darstellung von 1-Hexen durch die Katalysatorvorstufe und Methylaluminoxan selektiv katalysiert wird, und wobei die Katalysatorvorstufe einfacher herzustellen ist. Insbesondere soll die Herstellung der Vorstufe auf möglichst wenig toxischen Ausgangsstoffen beruhen. Der Katalysator soll außerdem bei hoher Reaktivität eine lange Standzeit aufweisen.

Die Erfindung wird durch die Darstellung von 1-Hexen gemäß dem ersten Patentanspruch und durch die Katalysatorvorstufe gemäß dem zweiten Patentanspruch gelöst.

Erfindungsgemäß wird für die Umsetzung von Ethen zu 1-Hexen ein Katalysator mit der in Anspruch 1 dargestellten Strukturformel eingesetzt, in der Y entweder Phosphor oder Schwefel darstellt und R Alkyl- oder Arylreste sind. Unter Alkyl- oder Arylresten sind sowohl die unsubstituierten als auch substituierte Substituenten zu verstehen. Alkylreste können verzweigt oder unverzweigt sein und eine Kohlenstoffzahl von 1 bis 10 aufweisen. In gleicher Weise können Arylreste substituiert sein, beispielsweise durch unterschiedlich lange, verzweigte oder unverzweigte Alkylketten oder durch Cycloalkyle wie z. B. Cyclohexyl. Alkylketten können auch ungesättigte Kohlenstoff-Kohlenstoff-Bindungen aufweisen.

Besonders bevorzugt werden die beiden folgenden Verbindungen als Katalysatorvorstufen:

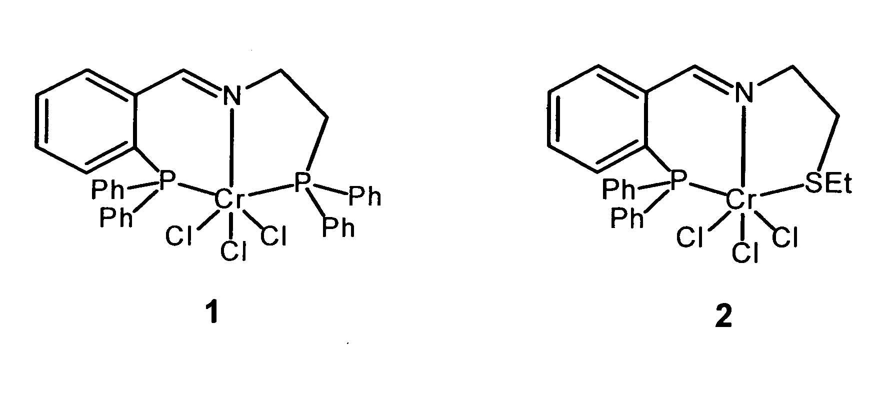

Die Herstellung der Katalysatorvorstufe kann über die folgende Reaktion erfolgen: 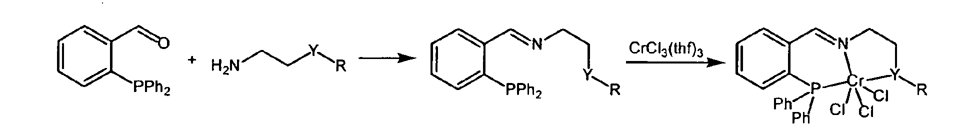 Auf diese Weise ist die Katalysatorvorstufe einfacher darstellbar als die bekannten Katalysatorvorstufen.

Die Erfindung wird im Folgenden anhand von Versuchsbeispielen näher erläutert.

### Darstellung der Imin-Liganden:

Unter Argon werden 3 mmol des Aldehyds in 40 mL abs. CH₂Cl₂ vorgelegt. Dazu werden zügig 3 mmol des Amins unter Rühren zugegeben. Die Reaktionsmischung wird 4 Stunden bei Raumtemperatur gerührt und anschließend das Lösungsmittel im Vakuum entfernt. Der ölige Rückstand wird aus Hexan in der Kälte kristallisiert und man erhält das Imin als farblosen bis gelben Feststoff in 40-80 % Ausbeute.

### Darstellung der Katalysatorvorstufe (Chrom(III)-Komplexe):

5 mmol CrCl₃(thf)₃ werden in 20 mL THF gelöst. Eine Lösung von 5 mmol des Imin- oder Aminliganden in 10 mL abs. THF werden zügig unter Rühren zu der roten Chromchloridlösung zugegeben. Man rührt 12 Stunden bei Raumtemperatur. Das Lösungsmittel wird im Vakuum entfernt und man erhält einen pulvrigen Rückstand des Chromkomplexes. Die Ausbeuten betragen 80-95 %.

### Durchführung der Katalyseversuche bei 30 bar Ethenüberdruck:

In einem Schlenkkolben werden 10 mmol der Chrom(III)-diert. Ein 150 mL Stahlautoklav wird evakuiert und mehrmals mit Argon gespült. Die Lösung mit dem Präkatalysator wird dann unter Argon in das Reaktionsgefäß gegeben. Unter Rühren werden 0.6 mL MAO (Methylaluminoxan) zugegeben. Das Autoklavengefäß wird verschlossen und mit 30 bar Ethen befüllt. Der Druck wird über die Dauer der Reaktion durch manuelle Zuführung von Ethen konstant gehalten. Zum Abbruch der Reaktion wird vorsichtig belüftet und die Reaktionslösung mit verdünnter Salzsäure und Wasser versetzt. Danach erfolgt die quantitative GC-Analyse der organischen Phase.

### Durchführung der Katalyseversuche bei 3 bar Ethenüberdruck:

Gleiche Durchführung wie bei den oben beschriebenen Versuchen. Nach Verschließen des Reaktors wird das Gefäß mit 3 bar Ethen befüllt.

| Nr. | Präkatalysator Menge (µmol)^{a} | Ethenüberdruck (bar) | Temperatur (°C) | Laufzeit (h) | PE (wt%) | Hexen (wt%) | α-Selektivität des Hexens | Produktivität (h⁻¹) |
|---|---|---|---|---|---|---|---|---|
| 1 | 1 (10) | 30 | 24-30 | 1 | 17 | 83 | 98 | 5742 |
| 2 | 1 (10) | 3 | 24 | 2 | 3 | 97 | 99 | 470 |
| 3 | 2 (10) | 30 | 24-30 | 1 | 18 | 82 | 99 | 2268 |

| | | | | | | | | |
|---|---|---|---|---|---|---|---|---|
| ^{a} 100 eq. MAO als Cokatalysator | | | | | | | | |

In der Tabelle sind die Ergebnisse der Darstellung von 1-Hexen unter Verwendung der Katalysatorvorstufen 1 und 2 sowie MAO zusammengefasst. Dabei bedeuten:
n_{Kat}: Stoffmenge an eingesetzter Katalysatorvorstufe
t: Reaktionszeit in Stunden
p: Reaktionsdruck in Bar
T: Temperatur in °C
Umsatz: Gesamtumsatz gebildeten Hexens
TOF: "Turn over frequency": Umsatzrate pro Zeiteinheit
PE: die Ausbeute an Polyethylen in Gewichtsprozent
Hexen: die Ausbeute an Hexen in Gewichtsprozent
% α-Olefin: die Ausbeute an α-Olefinen insgesamt.

## Patentansprüche

1. Verfahren zur Darstellung von 1-Hexen, bei dem
- Ethen
- in Gegenwart einer Katalysatorvorstufe, bei der Chrom mit einem Liganden, der Phosphor und Stickstoff enthält, komplexiert ist, und
- in Gegenwart von Methylaluminiumoxan als Co-Katalysator
- zu 1-Hexen umgesetzt wird,
**dadurch gekennzeichnet, dass** die Katalysatorvorstufe durch die folgende chemische Formel beschrieben wird, 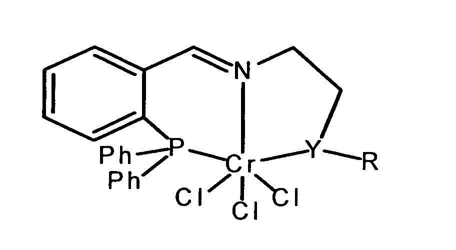 wobei Y entweder Phosphor oder Schwefel darstellt und R entweder Aryl-oder Alkylreste sind.

2. Katalysatorvorstufe zur Katalyse der Darstellung von 1-Hexen aus Ethen mit der folgenden chemischen Formel: 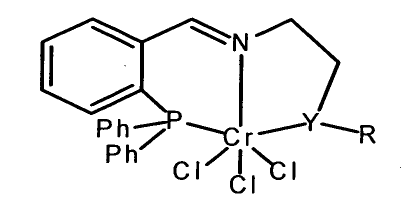 wobei Y entweder Phosphor oder Schwefel darstellt und R entweder Aryl- oder Alkylreste sind.
